# EUROPEAN PATENT SPECIFICATION

(11) **EP 3 915 568 B1**
(45) Date of publication and mention of the grant of the patent: **14.10.2026**
(21) Application number: 21176689.4
(22) Date of filing: 28.05.2021
(51) Int. Cl.: A61K 31/728, A61K 31/58, A61K 31/573, A61P 1/02

(54) **MUCOADHESIVE COMPOSITION FOR USE IN THE TREATMENT OF ORAL WOUND**
MUCOADHÄSIVE ZUSAMMENSETZUNG ZUR VERWENDUNG BEI DER BEHANDLUNG VON ORALEN WUNDEN
COMPOSITION MUCOADHÉSIVE À UTILISER DANS LE TRAITEMENT DES PLAIES BUCCALES

(30) Priority: 29.05.2020 IT 202000012853
(43) Date of publication of application: 01.12.2021
(73) Proprietor: Piccolo Federico, 80038 Pomigliano d'Arco (NA) (IT)
(72) Inventor: DESIDERIO, Vincenzo, 80137 Napoli (NA) (IT); CARAGLIA, Michele, 83031 ARIANO IRPINO (AV) (IT); PEPE, Giuseppe, 80011 ACERRA (NA) (IT); LAINO, Luigi, 80121 Torre del Greco (NA) (IT); PICCOLO, Federico, 80038 Pomigliano d'Arco (NA) (IT)
(74) Representative: Cattaneo, Elisabetta

(56) References cited:
- WO-A1-2011/110225
- WO-A1-2014/110454
- WO-A1-2017/120125
- US-A- 5 972 906
- ANONYMOUS: "KENALOG in ORABASE - NEW ZEALAND DATA SHEET NEW ZEALAND DATA SHEET", 5 April 2018 (2018-04-05), XP055774768, Retrieved from the Internet <URL:https://www.medsafe.govt.nz/profs/datasheet/k/kenaloginOrabasepaste.pdf> [retrieved on 20210210]
- JH LEE ET AL: "The efficacy of topical 0.2%hyaluronic acid gel on recurrent oral ulcers", JOURNAL OF THE EUROPEAN ACADEMY OF DERMATOLOGY AND VENEREOLOGY : JEADV NOV 2009,, vol. 22, no. 5, 1 May 2008 (2008-05-01), pages 590 - 595, XP009502595, ISSN: 1468-3083, [retrieved on 20071217], DOI: 10.1111/J.1468-3083.2007.02564.X
- NIZIC LAURA ET AL: "Innovative sprayablein situgelling fluticasone suspension: Development and optimization of nasal deposition", INTERNATIONAL JOURNAL OF PHARMACEUTICS, vol. 563, 6 April 2019 (2019-04-06), pages 445 - 456, XP085671085, ISSN: 0378-5173, DOI: 10.1016/J.IJPHARM.2019.04.015

## Description

### FIELD OF THE INVENTION

The present invention is related to pharmaceutical composition for use in the treatment of post-surgical wounds after different types of surgical interventions to the mucosal wall and/or tooth, recessions of teeth and implants, non-contagious mouth ulcers (aphthae). In a preferred embodiment the invention is a gel product of the pharmaceutical composition of the invention.

### STATE OF THE ART

Wound healing is a complex and dynamic process, which is still far to be completely understood. Wounds can be classified according to different characteristics such as trigger factor, exposure to the external environment, wound depth, the time frame of healing, potential risk of infections (Chhabra et al., 2017). Two main processes are involved in wound healing: regeneration and repair.

Regeneration involves the activation of stem cells able to reconstitute the integrity of the tissue which will be indistinguishable from the tissue before the injury. On the contrary, the repair involves the formation of a fibrotic scar tissue in which the architecture and functionality of the original tissue are partially lost.

Wound healing can be classified into three different categories: by primary, secondary or tertiary intention. Wound healing by primary intention happens when there is minimal tissue loss, e.g. wound sutured or taped. This wound will heal with a thin and clean scar, low inflammation, edema, and pain. Wound healing by secondary intention occurs when part of the tissue is lost and there is a much larger defect. In this case, the reparative process is much more complicated involving greater inflammation, edema, and pain prolonged for a longer time compared to primary intention wound healing. Wound healing by tertiary intention occurs when a process by secondary intention is intentionally interrupted and the wound will be mechanically closed, e.g after granulation tissue has formed.

Despite advances in non-surgical diagnostic techniques, partial or total excision of oral benign, premalignant and malignant disorders is needed to obtain a final histological diagnosis (Carreras-Torras & Gay-Escoda, 2015). Harvesting of a proper amount of tissue from the lesion allows the pathologist an easier and safer histological examination (Macey et al., 2015). For some diseases, total excision of the lesion should be obtained in order to decrease the possibility of relapse (Bahadure et al., 2012). Scalpel biopsy of widespread lesions results in a big amount of collected tissues and exposes the wound to the possibility of healing by secondary intention (Stell et al., 1982). Epithelial cells need some days to migrate from the surrounding tissues to the wound site, and in this period the connective donor tissues are exposed to the oral cavity environment (Singer & Clark, 1999). This is associated with higher morbidity for patients if compared to the healing by first intention (Eisen, 1992). Aphtha, another disease occurring in the mouth, is also named as "aphthous ulcer" and is an ulcer formation with pain occurring in the mouth. A typical aphtha sore is observed as red oval restricted sores on the mucosa layer in the mouth. Recurrent aphthous ulcer (RAU) is one of the most common ulcerative diseases of the oral mucosa, which is recurrent, painful and slow to heal. Many strategies and products have been proposed in order to improve wound healing, reduce pain and morbidity for the patients such as wound dressing, growth factors, skin substitutes, collagens dressing, topical insulin, antioxidant, hyperbaric chamber, etc (Chhabra et al., 2017). KENALOG in ORABASE oral paste 1mg/g (New Zealand data sheet) discloses composition for the treatment of oral lesions comprising triamcinolone acetonide in an adhesive vehicle which ensures a long contact of the active agent with the oral lesion. US5972906 discloses composition comprising hyaluronic acid and NSAID in the treatment of mucous membrane trauma. WO2017/120125 discloses composition comprising corticosteroids for the treatment of oral inflammatory lesions and ulcerative lesions in the treatment of aphthous ulcers and lichen planus.

Despite these attempts, there is still the need for appropriate treatments of wounds, especially regarding the secondary and tertiary healing wounds in the oral cavity.

In this context, the use of corticosteroids was normally described as detrimental for the healing as they reduce wound contraction and inflammation (Wang et al., 2013). Nevertheless, dosage and timing seem to play a very important role in determining the effect of steroids on the healing. Indeed, pretreatment with methylprednisolone has been used to reduce post-surgical complications after third molar extraction (Esen et al., 1999), as well as topical steroids, which were used to treat chronic ulcers (Hofman et al., 2007).

Moreover, buccal application of bilayer mucoadhesive films loaded with only 1 mg of prednisolone provided mucoadhesive and convenient application and was able to promote recurrent aphthous ulcer healing with shorter treatment duration (Farid & Wen, 2017)

Hyaluronic acid (HA) exerts an anti-inflammatory effect during oral wound-healing and is commonly applied after tooth extraction. Generally, previous studies about the subject have focused on tissue inflammatory responses after an extrinsic HA application. It has been hypothesized that HA of appropriate consistency increases also cell motility (Aya & Stern, 2014). It is also well known that topical administration of HA can be used in the postoperative treatment of patients undergoing dental procedures with positive results also in chronic gingival inflammation, periodontal diseases and oral ulcers (Casale et al., 2016)

In recent years, many adhesive mucosal dosage forms including primarily adhesive tablets, gels, patches, and polymeric films have been developed (Perioli et al., 2004). The expected features from buccal drug release systems are flexible and good bioadhesive properties and as such to ensure the keeping of the drug in the oral cavity for a desired period. Moreover, the therapeutic response required is provided by means of the release of the drug in a controlled and predictable manner by buccal systems in mucoadhesive structure (Wong et al., 1999). In recent years, controlled drug release technologies are increasingly gaining importance. The reduction in dose frequency, enhancement in patient compliance, minimization of side effects, acquisition of longer-term effects and reduction of treatment costs are among the advantages of these systems (Wise, 2000)

However, there is still the need for appropriate treatments for wounds, especially in regard to secondary and tertiary healing wounds in the oral cavity.

The object of the present invention is hence to provide a treatment for wounds in the oral cavity, especially for more severe cases such as secondary and tertiary healing wound.

### SUMMARY OF THE INVENTION

The inventors noted that corticosteroids, normally described as detrimental for wound healing, when associated with hyaluronic acid, preferably in certain concentrations, have a positive effect on wound healing.

In view of the above the inventors realized that they can provide a pharmaceutical composition for use in the treatment of a post-surgical wound, said composition comprising hyaluronic acid or a salt thereof together with at least one corticosteroid and a mucoadhesive polymer as suitable vehicle for the intended oral use.

Furthermore, the inventors found out that specific weight ratios of essential ingredients were able to guarantee the healing of a post-surgical wound.

Therefore, the present invention relates to a pharmaceutical composition comprising:
i. a mucoadhesive polymer;
ii. hyaluronic acid or a pharmaceutically acceptable salt thereof, and
iii. at least one corticosteroid

for use in the treatment of a post-surgical wound,
wherein the weight ratio of the components i.: ii. is in the range from 3:2 to 8:3.

The amount of corticosteroid can be varied, and will be an efficacious dose within the composition, preferably in the range from 0.005 to 10%, more preferably 0.01 to 5% by weight of the total amount of the pharmaceutical composition.

In the case of clobetasol propionate as component iii as corticosteroid the range will be from 0.025 to 0.05% by weight of the total amount of the pharmaceutical composition.

Ratios among the components different than what it is claimed will affect the efficacy of the formulation.

In fact, the inventors deem that lower amounts of mucoadhesive polymer will not allow for a necessary viscosity and adherence to oral mucosa of the composition of the invention and hence of the film necessary to protect the wounds and promote the healing and lower amounts of HA will not allow a proper hydration of the tissue and the stimulation of the endogenous physiological process that speeds up the tissue regeneration. On the other hand, higher amounts of mucoadhesive polymer will increase the viscosity of the formulation of the invention, thus hindering the proper placement of the gel on the wound and higher amounts of HA will not stimulate the physiological process needed for tissue regeneration and will not allow a proper delivery of the corticosteroid.

Lower amounts of corticosteroid with respect to the preferred ones will not permit the control of inflammation, edema and pain and higher amounts of corticosteroid will be detrimental for the wound and could create safety issue as it could be absorbed systemically.

The three components and the ratio between components i. and ii. were all essential for promoting the surgical wound healing.

In a preferred advantageous embodiment, the pharmaceutical composition of the invention is in the form of a gel product.

The present invention hence relates to a pharmaceutical gel formulation comprising the pharmaceutical composition according to the invention and suitable additives for use in the treatment of post-surgical wounds.

The gel product here described is formulated by combining three primary components: i. mucoadhesive polymer; ii. hyaluronic acid iii. at least a corticosteroid according to the claimed composition.

Advantageously, once applied to the oral mucosa the gel product adheres to the tissues and forms a protective film, the active components promote wound healing, reduce inflammation, edema, and pain.

In a preferred form of invention, the mucoadhesive polymer is sodium carboxymethyl cellulose (CMC). The inventors realized that CMC has important mucoadhesive properties even if compared to other materials and has a good releasing time of lipophilic drugs including corticosteroids such as prednisolone.

Therefore, the current invention is advantageously provided in the form of a mucoadhesive gel product preferably comprising hyaluronic acid or its pharmaceutically acceptable salt, carboxymethylcellulose as a mucoadhesive polymer, and a corticosteroid in specific weight ratios between hyaluronic acid or its pharmaceutically acceptable salt and carboxymethylcellulose.

Specifically, this gel formulation applied to post-surgery wounds is able to promote wound healing, reduce edema and pain thus reducing morbidity for the patient.

Without being bound to any theory the inventors deem that the combination of the invention of using corticosteroids and mucoadhesive polymer with hyaluronic acid in the given amounts resulted to be a surprising finding. Indeed, corticosteroids have been widely described as detrimental for wound healing while, in this invention, the combination with HA resulted in an improvement of the healing.

Moreover, the presence of both CMC and HA was useful to grant optimal mucoadhesive properties and allowed the controlled slow release of the corticosteroids in situ where the lesion of the oral mucosa is placed.

### DESCRIPTION OF THE FIGURES

Figure 1 shows the effect of the use of the pharmaceutical composition of example 2;
Figure 2 shows the effect of the use of the pharmaceutical composition of example 3; and
Figure 3 shows the effect of the use of the pharmaceutical composition of example 4.

### DETAILED DESCRIPTION OF THE INVENTION

The invention regards a pharmaceutical composition comprising:
i. a mucoadhesive polymer;
ii. hyaluronic acid or a pharmaceutically acceptable salt thereof, and
iii. at least one corticosteroid

for use in the treatment of a post-surgical wound,
wherein the weight ratio of the components i.: ii. is in the range from 3:2 to 8:3.

The amount of corticosteroid can be varied, and will be an efficacious dose within the composition, preferably in the range from 0.005 to 10%, more preferably 0.01 to 5% by weight of the total amount of the pharmaceutical composition.

The pharmaceutical composition for use of the invention comprises a mucoadhesive polymer in an amount in the range from 3 to 8% w/w with respect to the total amount of the composition. Preferably that amount is in the range from 3 to 7%w/w, more preferably 3.2 to 6% by weight with respect to the total weight amount of the composition.

Mucoadhesive polymers can be preferably selected from the group consisting of:
- cellulose derivatives such as methyl-cellulose, ethyl-cellulose, hydroxyethyl-cellulose, hydroxypropyl-cellulose, hydroxypropyl-methylcellulose, sodium carboxymethylcellulose,
- polyacrylic acid polymers such as carbomers and polycarbophils, poly(hydroxyethyl-methacrylate),
- poly(ethylene-oxide),
- poly(vinyl pyrrolidone),
- poly(vinyl alcohol),
- sodium alginate,
- karaya gum,
- guar gum,
- xanthan gum,
- gelatine,
- pectin and chitosan and
combinations thereof.

In a preferred embodiment of the invention, mucoadhesive polymer used in the formulations is sodium carboxymethylcellulose.

The pharmaceutical composition for use of the invention comprises at least one corticosteroid, preferably in the range from 0.005 to 1% by weight of the total amount of the pharmaceutical composition, more preferably in an amount in the range from 0.015 to 0.07% w/w with respect to the total weight amount of the composition. In a still more preferred embodiment, that amount is in the range from 0.02 to 0.06%w/w, more preferably 0.02 to 0.05% by weight with respect to the total weight amount of the composition.

The at least one corticosteroid of the pharmaceutical composition is preferably selected from the group consisting of hydrocortisone, cortisone, tixocortol, prednisolone, prednisone, methyl-prednisolone, triamcinolone, mometasone, amcinonide, budesonide, desonide, fluocinonide, fluosinilo, halcinonide, betamethasone, dexamethasone, fluocortolone, beclomethasone, beclomethasone acetate, clobetasol e clobetasol propionate.

In a preferred embodiment the at least one corticosteroid in the composition is clobetasol propionate or its salt. In the case of clobetasol propionate as preferred component iii. as corticosteroid the amount range will be from 0.025 to 0.05% by weight of the total amount of the pharmaceutical composition.

The pharmaceutical composition for use according to the invention comprises hyaluronic acid or a pharmaceutically acceptable salt in an amount in the range from 2 to 3% w/w with respect to the total weight amount of the composition. Preferably that amount is in the range from 2 to 2.5%w/w, more preferably 2 to 2.2% by weight with respect to the total weight amount of the composition.

Preferably hyaluronic acid is in the form of the sodium salt.

With hyaluronic acid in the present invention it is intended hyaluronic acid having IUPAC name (3S,4R,6R)-3-[[(2R,4R,5S)-3-acetamido-4-[[(2R,4R,5S)-6-carboxy-3,4,5-trihydroxyoxan-2-yl]oxymethyl]-5-hydroxy-6-(hydroxymethyl)oxan-2-yl]methoxymethyl]-6-[[(2R,4R,5S)-3-acetamido-2,5-dihydroxy-6-(hydroxymethyl)oxan-4-yl]methoxy]-4,5-dihydroxy-5-methyloxane-2-carboxylic acid in polymeric form with preferably molecular weight in the range from 800,000 to 4,000,000 Dalton, more preferably in the range from 1,000,000 to 2,000,000 Dalton.

In a preferred advantageous embodiment, the pharmaceutical composition of the invention is in the form of a gel product.

The pharmaceutical composition according to the invention hence can comprise pharmaceutically acceptable excipients so as the composition can be administered as a gel formulation.

The pharmaceutically acceptable excipients are preferably at least one surface-active agent and/or at least one in situ gelling polymer.

The present invention hence relates to a pharmaceutical gel formulation comprising the pharmaceutical composition according to the invention and pharmaceutically acceptable excipients for use in the treatment of a post-surgical wound.

The present disclosure relates to a pharmaceutical gel formulation comprising the pharmaceutical composition according to the invention and pharmaceutically acceptable excipients for use in the treatment of recurrent aphthous ulcers or Recurrent aphthous stomatitis (RAS).

The pharmaceutical gel formulation comprises as pharmaceutically acceptable excipient preferably at least one surface-active agent and/or at least one in situ gelling polymer. Said surface-active agents can be more preferably selected from a group consisting of Tween 80, ascorbic palmitate and Cremophor RH 40 and combinations thereof.

In a preferred advantageous embodiment, therefore the pharmaceutical formulation of the invention is in the form of a gel product.

The present invention hence relates to a pharmaceutical gel formulation comprising the pharmaceutical composition according to the invention and suitable additives for use in the treatment of a post-surgical wound.

The pharmaceutical gel formulation for use of the invention is a gel product; that, once applied to the oral mucosa, adheres to the tissues and forms a protective film, the active components promote wound healing, reduce inflammation, edema, and pain.

The current invention is advantageously provided in the form of a mucoadhesive gel product preferably comprising sodium hyaluronic acid, carboxymethylcellulose as a mucoadhesive polymer, and clobetasol propionate.

Specifically, this pharmaceutical formulation of the invention applied to post-surgery wounds is able to promote wound healing, reduce edema and pain thus decreasing morbidity for the patient.

The present invention relates to a pharmaceutical composition and a pharmaceutical gel formulation for use in the treatment of oral post-surgical wounds. The pharmaceutical gel formulation can be applied on the buccal mucosa, for wound healing after oral surgical intervention. As surgical intervention extraction of the third molar can be cited.

The pharmaceutical gel formulation can be applied on the buccal mucosa, preferably for the treatment of oral aphthae lesions.

Advantageously the presence of three different components (corticosteroid, hyaluronic acid or its pharmaceutically acceptable salt and a mucoadhesive polymer, preferably carboxymethylcellulose.), in the specific amounts, accelerates the wound healing inducing fibroblast differentiation and proliferation through a local slow release of corticosteroids and contact with HA through mucoadhesive properties of the mucoadhesive polymer, preferably CMC.

Advantageously, the composition and the formulation according to the invention guarantees the contact of an active agent for a long time with the mucosa by means of the system that allows adhesion for a long time and release of active drug in a long and sustained manner.

The current invention prolongs the retention time in the mouth of the formulations if compared to previous medications that were in form of pastilles or oral suspensions The invention will be now detailed by non-limitative examples.

### EXPERIMENTAL PARTS.

### Example 1:

### Preparation of the gel formulation of the invention (formulation 1)

The formulation was prepared in two phases as follows:
Phase A (% Vol) comprised: purified water 92%, potassium sorbate 0.5%, sodium benzoate 0.5%, EDTA 0.10 %, propylene glycol 1%, sodium hyaluronate oral grade 2.2%, carboxymethyl cellulose 3.2%, Tego Carbomer 140 0.5%
Phase B (% Vol) comprised: Zetemuls COH40 (cationic surfactant comprising PWG-40 Hydrogenated Castor Oil) 5.7% Caprylic capric triglycerides 70%, phenoxyethanol 23%, clobetasol (9α-Fluoro-11β,17α-dihydroxy-16β-methyl-21-chloropregna-1,4-diene-3,20-dione) propionate 0.05 %.
Phase A and Phase B were independently prepared, and B was mixed in A in a ratio of 1/20.

### Example 2

### Effects of the formulation of the invention

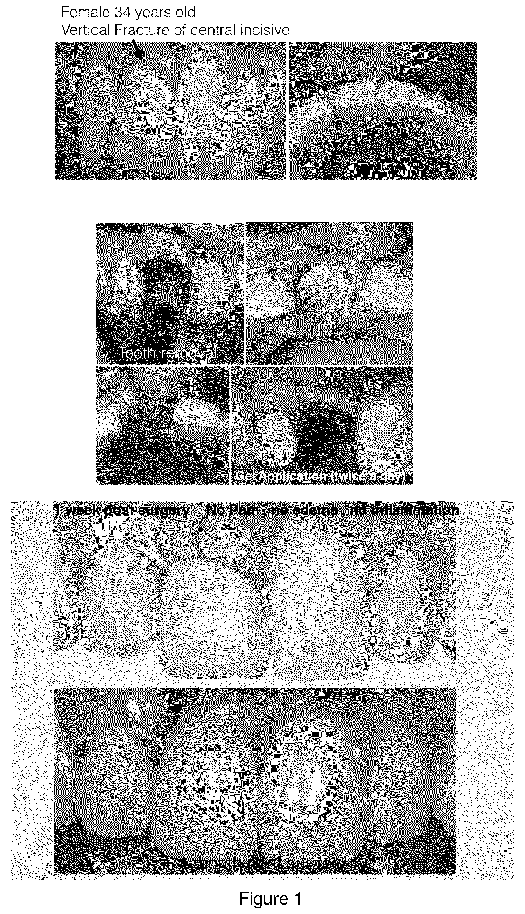

The formulation as prepared and reported in Example 1 was applied on the buccal mucosa of a 34 years old female with a vertical fracture of the central incisive. The tooth was removed and the gel was applied twice a day. After 5 days from the surgery no pain, edema and inflammation were observed, and similar effects were still recorded at 1 month from the intervention. The results are evident from Figure 1.

### Example 3.

### Effects of the formulation of the invention

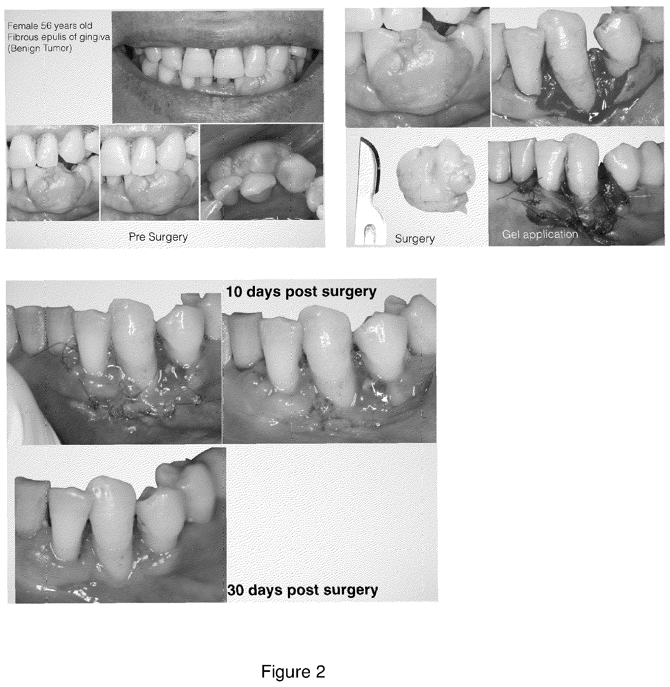

The formulation as prepared and reported in Example 1 was applied on the buccal mucosa of a 56 years old female with a fibrous epulis of gingiva (benign tumour). The tumour was removed and the gel was applied on the surgical wound twice a day. After 10 days from the surgical intervention no edema, pain and inflammation were observed, and similar effects were still recorded at 1 month from the intervention. The results are evident from Figure 2.

### Example 4.

### Effects of the formulation 1 of the invention

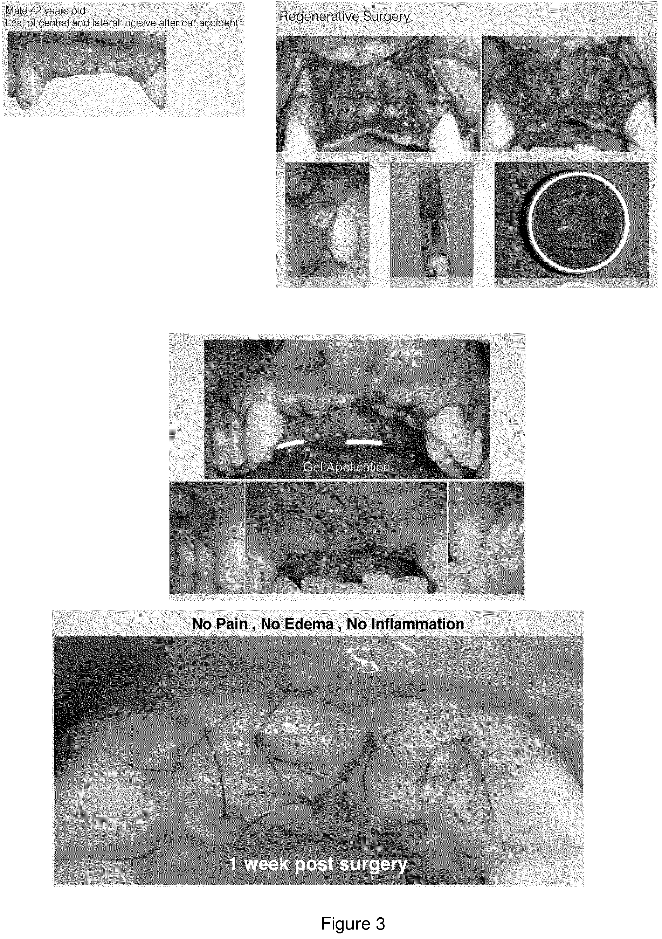

The formulation as prepared and reported in Example 1 was applied on the buccal mucosa of a 42 years old male with loss of central and lateral incisive after car crash. Regenerative surgery was performed, and gel was applied on the surgical wound twice a day. After 5 days from the surgical intervention no edema, pain and inflammation were observed. The results are evident from Figure 3.

### Example 5

### Summary of the clinical effects on patients treated with formulation prepared as in the example 1.

28 patients were treated, their data are summarized in table 1.

| **Patients** | **Gender** | **Age** | **Diagnosis** | **Treatment** |
|---|---|---|---|---|
| G.D.G. | M | 52 | Aphta Major | |
| A.I. | M | 42 | Major Aphta | |
| A.R. | M | 29 | Major Aphta | |
| D.L. | M | 37 | Major Aphta | |
| T. D. | F | 34 | Major Aphta | |
| M.R. | M | 32 | Transversal Bone Defect | Regenerative Surgery |
| A.D.C. | F | 64 | Transversal Bone Defect | Regenerative Surgery |
| C.L. | F | 38 | Transversal Bone Defect | Regenerative Surgery |
| S.P. | M | 68 | Transversal Bone Defect | Regenerative Surgery |
| P.M. | M | 26 | Transversal Bone Defect | Regenerative Surgery |
| G.N. | F | 23 | Transversal Bone Defect | Regenerative Surgery |
| F.F. | M | 25 | Transversal Bone Defect | Regenerative Surgery |
| G.G | M | 64 | Transversal Bone Defect | Regenerative Surgery |
| M.P. | M | 45 | Transversal Bone Defect | Regenerative Surgery |
| E.E. | M | 55 | Transversal Bone Defect | Regenerative Surgery |
| D.C. | M | 31 | Transversal Bone Defect | Regenerative Surgery |
| G.Z. | M | 32 | Desquamative Gingivitis | |
| M.C. | F | 39 | Desquamative Gingivitis | |
| G.T | M | 46 | Desquamative Gingivitis | |
| E.G. | F | 66 | Desquamative Gingivitis | |
| D.R. | F | 23 | Desquamative Gingivitis | |
| O.N. | F | 60 | Erosive-Atrophic Oral Lichen Planus | |
| C.B. | M | 41 | Erosive-Atrophic Oral Lichen Planus | |
| R.S. | F | 62 | Erosive-Atrophic Oral Lichen Planus | |
| M.D.P. | F | 44 | Erosive-Atrophic Oral Lichen Planus | |
| L.I. | F | 56 | Erosive-Atrophic Oral Lichen Planus | |
| C.T. | F | 51 | Erosive-Atrophic Oral Lichen Planus | |
| M.T. | F | 18 | Erosive-Atrophic Oral Lichen Planus | |

5 patients had Major Aphtha, 11 patients presented a transversal bone defect and were treated with regenerative surgery, 5 patients were affected by Desquamative Gingivitis; 7 patients had Erosive-Atrophic Oral Lichen Planus.

All patients underwent the following protocol: application of the product every 12 hours on the affected site for 15 days. The follow-ups were carried out in 5 days, 10 days and 15 days.

The parameters observed were: decrease in pain and burning symptoms, timing and quality of clinical healing, reduced inflammation of the treated tissues.

A significant reduction in symptoms, acceleration of healing times, healing of the treated site with the absence of post-operative complications in patients undergoing regenerative surgery and reduction of inflammatory clinical parameter were found in all patients.

### Example 6:

### Preparation of the gel formulation of the invention (formulation 2)

The following formulation was prepared:
Hyaluronic acid 2.2%, Clobetasol (9α-Fluoro-11β,17α-dihydroxy-16β-methyl-21-chloropregna-1,4-diene-3,20-dione) propionate 0.05%, carboxymethylcellulose 6.0%, polysorbate 20 0.1%, potassium sorbate 0.2%, sodium edetate 0.01%, sodium citrate dihydrate, citric acid monohydrate until pH is 4.5 - 5.5, water to 100%.

This formulation was prepared in 5 steps (10 liters batch):
Step 1: 8L of water and 0.22 Kg of hyaluronic acid were homogenized to reach a uniform gel;
Step 2: in a separated container 0.6L of water with 0.6 Kg of carboxymethylcellulose were homogenized. The solution so obtained has been added to the gel obtained in step 1 and the homogenization was continued for 15 minutes.
Step 3: the following components were then added to the gel:
   - 0.01 Kg of polysorbate 20, and the homogenization was continued for 15 minutes
   - 0.11 Kg clobetasol propionate very slowly and the homogenization was continued until a complete dissolution was obtained.
Step 4: in a separate container 0.4 L of water was added and then one by one were added:
   0,02 Kg of potassium sorbate
   0,001 Kg of sodium edetate
   0,0005 Kg of sodium citrate dihydrate.

The solution was then homogenized for 15 minutes
Step 5: the pH was verified and adjusted to 4,5-5,5 by adding citric acid monohydrate and the product was brought to final volume (10L) with water.

### Example 7:

### Evaluation of Formulation 2

This final product obtained in example 6 (formulation 2) was used on 10 patients with transversal bone defect that were treated with regenerative surgery. 10 more patients undergoing regenerative surgery were not treated and considered as control group.

All patients underwent the following protocol: application of the product every 12 hours on the affected site for 7 days followed by one application every 24 hours for additional 14 days. The follow-ups were carried out at 2,5, 8, 14, 21, and 28
days.

The parameters observed were:
- quality of wound healing,
- reduced inflammation of the treated tissues
- Improvement in symptom severity,

### Quality of wound healing and inflammation

Clinical Healing score (clinical healing scores a sum of 5 - criteria) which included evaluation of redness, edema, suppuration, healthy granulation tissue, and signs of epithelialization [Excellent Healing (Score 0), Very good healing (Score 1), Good healing (Score 2), Adequate healing (Score 3), Poor healing (Score 4) and No healing (Score 5)]. (Mahajan 2018). This parameter was evaluated on day 7, 14 and 28.

### Measurements of wound closure by digital photographs

The wound area was calculated using ImageJ software (NIH, USA). Morphometric analysis of the wounds was performed using images of the wounds at 0, 5, 8, 14, 21, and 28 days after the wound. The wound closure rate representing the percentage of wound reduction from the original wound size will be calculated using the following formula: ((wound area on day 0 - wound area on days 5, 8, 14, 21, and 28) / wound area day 0) × 100. Values were expressed as a percentage of wounds healed. (Guidoni, 2019)

### Improvement in symptom severity.

Pain was evaluated with a Numeric Rating Scale (NRS) on days 2, 5, 8, and 14. The NRS requires the patient to rate their pain on a defined scale of 0-10 where 0 is no pain and 10 is the worst pain imaginable (Palaia 2019) as follows:
Value 0= no pain
Values 1,2,3= mild pain
Values 4,5,6= moderate pain
Values 7,8,9= severe pain

A significant reduction in symptoms, acceleration of healing times, healing of the treated site with the absence of post-operative complications in patients undergoing regenerative surgery and reduction of inflammatory clinical parameters were found in all patients.

The average of Clinical Healing score in control group vs treatments group was as follows:
- day 7: 4,2 vs 3,5
- day 14: 3,8 vs 1,8
- day 28: 2,1 vs 1,6
the average morphometric analysis of the wound values in control group vs treatments group were as follows:
- day 5: 21,5% vs 35,4%
- day 7: 26,2% vs 41,3%
- Day 14: 56,6% vs 81,2%
- Day 21: 87,7% vs 98,1%
- Day 28: 97,4% vs 99,4%

The average of Numeric Rating Scale (NRS) in control group vs treatments group was as follows:
- day 2: 7,2 vs 5,1
- day 5: 5,3 vs 3,1
- day 7: 4,2 vs 2,7
- day 14: 2,8 vs 1,1

Therefore the pharmaceutical composition of the invention was efficacious in improving the wound healing process, reduce pain and inflammation in patients that were treated with regenerative surgery.

## Claims

1. A pharmaceutical composition comprising:
i. a mucoadhesive polymer;
ii. hyaluronic acid or a pharmaceutically acceptable salt thereof, and
iii. at least one corticosteroid
for use in the treatment of an oral post-surgical wound,
wherein the weight ratio of the components i.: ii. is in the range from 3:2 to 8:3.

2. The pharmaceutical composition for use of claim 1, wherein the amount of corticosteroid is in the range from 0.005 to 10%, preferably 0.01 to 5% by weight of the total amount of the pharmaceutical composition.

3. The pharmaceutical composition for use of claim 2, wherein the amount of corticosteroid is in the range from 0.015 to 0.07% w/w with respect to the total weight amount of the composition, preferably 0.02 to 0.06%w/w, more preferably 0.02 to 0.05% by weight with respect to the total weight amount of the composition.

4. The pharmaceutical composition for use according to anyone of claims 1-3, wherein the mucoadhesive polymer is selected from the group consisting of: cellulose derivatives such as methyl-cellulose, ethyl-cellulose, hydroxyethyl-cellulose, hydroxypropyl-cellulose, hydroxypropyl-methylcellulose, sodium carboxymethylcellulose; polyacrylic acid polymers such as carbomers and polycarbophils, poly(hydroxyethyl-methacrylate); poly(ethyleneoxide); poly(vinyl pyrrolidone); poly(vinyl alcohol); sodium alginate; karaya gum; guar gum; xanthan gum; gelatin, pectin, chitosan and combinations thereof.

5. The pharmaceutical composition for use of claim 4, wherein the mucoadhesive polymer is sodium carboxymethylcellulose.

6. The pharmaceutical composition for use according to anyone of claims 1-5, wherein the at least a corticosteroid is selected from the group consisting of hydrocortisone, cortisone, tixocortol, prednisolone, prednisone, methyl-prednisolone, triamcinolone, mometasone, amcinonide, budesonide, desonide, fluocinonide, fluosinilo, halcinonide, betamethasone, dexamethasone, fluocortolone, beclomethasone, beclomethasone acetate, clobetasol and clobetasol propionate.

7. The pharmaceutical composition for use according to claim 6, wherein the at least one corticosteroid in the composition is clobetasol propionate, preferably in the range from 0.025 to 0.05% by weight of the total amount of the pharmaceutical composition.

8. The pharmaceutical composition for use according to anyone of claims 1-7, wherein hyaluronic acid is in the form of the sodium salt.

9. The pharmaceutical composition for use according to anyone of claims 1-8, wherein hyaluronic acid has molecular weight in the range from 800,000 to 4,000,000 Dalton, preferably in the range from 1,000,000 to 2,000,000 Dalton.

10. The pharmaceutical composition for use according to anyone of claims 1-9 comprising pharmaceutically acceptable excipients to be administered as a gel formulation.

11. The pharmaceutical composition for use according to claim 10, wherein the pharmaceutically acceptable excipients are at least one surface-active agent and/or at least one in situ gelling polymer.

12. A pharmaceutical gel formulation comprising the pharmaceutical composition according to anyone of claims 1-11 and pharmaceutically acceptable excipients for use in the treatment of an oral post-surgical wound.

## Patentansprüche

1. Pharmazeutische Zusammensetzung, umfassend:
i. ein mucoadhäsives Polymer;
ii. Hyaluronsäure oder ein pharmazeutisch verträgliches Salz davon, und
iii. mindestens ein Kortikosteroid
zur Verwendung bei der Behandlung einer postoperativen oralen Wunde, wobei das Gewichtsverhältnis der Bestandteile i. : ii. im Bereich von 3 : 2 bis 8 : 3 liegt.

2. Pharmazeutische Zusammensetzung zur Verwendung nach Anspruch 1, wobei die Menge des Kortikosteroids im Bereich von 0,005 bis 10 Gew.-%, vorzugsweise 0,01 bis 5 Gew.-%, der Gesamtmenge der pharmazeutischen Zusammensetzung liegt.

3. Pharmazeutische Zusammensetzung zur Verwendung nach Anspruch 2, wobei die Menge des Kortikosteroids im Bereich von 0,015 bis 0,07 Gew.-%, bezogen auf das Gesamtgewicht der Zusammensetzung, vorzugsweise 0,02 bis 0,06 Gew.-%, noch bevorzugter 0,02 bis 0,05 Gew.-%, bezogen auf das Gesamtgewicht der Zusammensetzung, liegt.

4. Pharmazeutische Zusammensetzung zur Verwendung nach einem der Ansprüche 1-3, wobei das mucoadhäsive Polymer ausgewählt ist aus der Gruppe, bestehend aus: Cellulosederivaten wie Methylcellulose, Ethylcellulose, Hydroxyethylcellulose, Hydroxypropylcellulose, Hydroxypropylmethylcellulose, Natriumcarboxymethylcellulose; Polyacrylsäurepolymere wie Carbomere und Polycarbophile, Poly(hydroxyethylmethacrylat); Poly(ethylenoxid); Poly(vinylpyrrolidon); Poly(vinylalkohol); Natriumalginat; Karaya-Gummi; Guarkernmehl; Xanthan; Gelatine, Pektin, Chitosan und Kombinationen davon.

5. Pharmazeutische Zusammensetzung zur Verwendung nach Anspruch 4, wobei das mucoadhäsive Polymer Natriumcarboxymethylcellulose ist.

6. Pharmazeutische Zusammensetzung zur Verwendung nach einem der Ansprüche 1-5, wobei das mindestens eine Kortikosteroid aus der Gruppe ausgewählt ist, bestehend aus Hydrocortison, Cortison, Tixocortol, Prednisolon, Prednison, Methylprednisolon, Triamcinolon, Mometason, Amcinonid, Budesonid, Desonid, Fluocinonid, Fluosinilo, Halcinonid, Betamethason, Dexamethason, Fluocortolon, Beclomethason, Beclomethasonacetat, Clobetasol und Clobetasolpropionat.

7. Pharmazeutische Zusammensetzung zur Verwendung nach Anspruch 6, wobei das mindestens eine Kortikosteroid in der Zusammensetzung Clobetasolpropionat ist, vorzugsweise im Bereich von 0,025 bis 0,05 Gew.-% der Gesamtmenge der pharmazeutischen Zusammensetzung.

8. Pharmazeutische Zusammensetzung zur Verwendung nach einem der Ansprüche 1-7, wobei die Hyaluronsäure in Form des Natriumsalzes vorliegt.

9. Pharmazeutische Zusammensetzung zur Verwendung nach einem der Ansprüche 1-8, wobei die Hyaluronsäure ein Molekulargewicht im Bereich von 800.000 bis 4.000.000 Dalton, vorzugsweise im Bereich von 1.000.000 bis 2.000.000 Dalton, aufweist.

10. Pharmazeutische Zusammensetzung zur Verwendung nach einem der Ansprüche 1-9, umfassend pharmazeutisch verträgliche Hilfsstoffe, zur Verabreichung als Gelformulierung.

11. Pharmazeutische Zusammensetzung zur Verwendung nach Anspruch 10, wobei die pharmazeutisch verträglichen Hilfsstoffe mindestens ein Tensid und/oder mindestens ein in situ gelierendes Polymer sind.

12. Pharmazeutische Gelformulierung, umfassend die pharmazeutische Zusammensetzung nach einem der Ansprüche 1-11 und pharmazeutisch verträgliche Hilfsstoffe zur Verwendung bei der Behandlung einer postoperativen oralen Wunde.

## Revendications

1. Composition pharmaceutique comprenant :
i. un polymère mucoadhésif ;
ii. l'acide hyaluronique ou un sel pharmaceutiquement acceptable de celui-ci, et
iii. au moins un corticostéroïde
à utiliser dans le traitement d'une plaie post-chirurgicale orale, dans laquelle le rapport pondéral des composants i. : ii. est compris entre 3:2 et 8:3.

2. Composition pharmaceutique à utiliser selon la revendication 1, dans laquelle la quantité de corticostéroïde est comprise entre 0,005 et 10 %, de préférence entre 0,01 et 5 % en poids de la quantité totale de la composition pharmaceutique.

3. Composition pharmaceutique à utiliser selon la revendication 2, dans laquelle la quantité de corticostéroïde est comprise entre 0,015 et 0,07 % p/p par rapport à la quantité totale en poids de la composition, de préférence entre 0,02 et 0,06 % p/p, plus préférablement entre 0,02 et 0,05 % en poids par rapport à la quantité totale en poids de la composition.

4. Composition pharmaceutique à utiliser selon l'une quelconque des revendications 1 à 3, dans laquelle le polymère mucoadhésif est choisi dans le groupe constitué par : les dérivés de cellulose tels que la méthylcellulose, l'éthylcellulose, l'hydroxyéthylcellulose, l'hydroxypropylcellulose, l'hydroxypropylméthylcellulose, la carboxyméthylcellulose de sodium ; les polymères d'acide polyacrylique tels que les carbomères et les polycarbophiles, le poly(hydroxyéthylméthacrylate) ; le poly(éthylène-oxyde) ; la poly(vinylpyrrolidone) ; le poly(alcool vinylique) ; l'alginate de sodium ; la gomme de karaya ; la gomme de guar ; la gomme de xanthane ; la gélatine, la pectine, le chitosane et leurs combinaisons.

5. Composition pharmaceutique à utiliser selon la revendication 4, dans laquelle le polymère mucoadhésif est la carboxyméthylcellulose de sodium.

6. Composition pharmaceutique à utiliser selon l'une quelconque des revendications 1 à 5, dans laquelle le au moins un corticostéroïde est choisi dans le groupe constitué par l'hydrocortisone, la cortisone, le tixocortol, la prednisolone, la prednisone, la méthylprednisolone, la triamcinolone, la mométasone, l'amcinonide, le budésonide, le désonide, le fluocinonide, le fluosinilo, l'halcinonide, la bétaméthasone, la dexaméthasone, la fluocortolone, la béclométhasone, l'acétate de béclométhasone, le clobétasol et le propionate de clobétasol.

7. Composition pharmaceutique à utiliser selon la revendication 6, dans laquelle le au moins un corticostéroïde dans la composition est le propionate de clobétasol, de préférence entre 0,025 et 0,05 % en poids de la quantité totale de la composition pharmaceutique.

8. Composition pharmaceutique à utiliser selon l'une quelconque des revendications 1 à 7, dans laquelle l'acide hyaluronique est sous la forme du sel de sodium.

9. Composition pharmaceutique à utiliser selon l'une quelconque des revendications 1 à 8, dans laquelle l'acide hyaluronique a un poids moléculaire compris entre 800 000 et 4 000 000 Dalton, de préférence entre 1 000 000 et 2 000 000 Dalton.

10. Composition pharmaceutique à utiliser selon l'une quelconque des revendications 1 à 9, comprenant des excipients pharmaceutiquement acceptables à administrer sous forme de formulation en gel.

11. Composition pharmaceutique à utiliser selon la revendication 10, dans laquelle les excipients pharmaceutiquement acceptables sont au moins un agent tensioactif et/ou au moins un polymère gélifiant in situ.

12. Formulation de gel pharmaceutique comprenant la composition pharmaceutique selon l'une quelconque des revendications 1 à 11 et des excipients pharmaceutiquement acceptables à utiliser dans le traitement d'une plaie post-chirurgicale orale.
